# EUROPEAN PATENT SPECIFICATION

(11) **EP 2 687 174 B1**
(45) Date of publication and mention of the grant of the patent: **12.10.2016**
(21) Application number: 12382287.6
(22) Date of filing: 19.07.2012
(51) Int. Cl.: A61B 18/02, A61F 7/00

(54) **Head end for device for cool therapy**
Kopfende für Gerät für Therapie durch Kühlung
Tête de réseau pour appareil de thérapie par réfrigération

(43) Date of publication of application: 22.01.2014
(73) Proprietor: High Technology Products, SL, 08005 Barcelona (ES)
(72) Inventor: Sanchez Jaime Jose Antonio, 08037 Barcelona (ES)
(74) Representative: Molero Moraleda, Felipe

(56) References cited:
- WO-A2-2005/060354
- US-A1- 2007 255 362
- US-A1- 2008 269 851
- US-A1- 2008 287 839
- US-A1- 2010 280 582

## Description

Head for an apparatus for applying cold-based treatments of the type that comprises a chassis with an interior cavity, which houses the first cooling conductor plates, facing each other, at least one suction orifice connected to suction means, at least one Peltier cell for each first conductor plate in contact with this plate and with second conductor plates that dissipate heat towards cooling means, a temperature sensor that monitors the temperature of at least one Peltier cell and power supply means, further comprising an extractable, cold-conducting accessory with fixing means, placed inside the cavity, reducing the space of said cavity, with said accessory secured inside the cavity by the mentioned fixing means.

The invention is defined in the appended claims.

### BACKGROUND TO THE INVENTION

Various machines are known in the state of the art, which employ cold or cryotherapy as a method to reduce obesity and/or cellulitis.

Thus, Spanish Patent No 2300569 (EP1490005), "DEVICES FOR THE SELECTIVE INTERFERENCE OF FATTY TISSUE BY MEANS OF CONTROLLED COOLING", is known from 2003, in the name of THE GENERAL HOSPITAL CORPORATION, which refers to a device to selectively interfere with lipid-rich cells in humans, except small children, by cooling that comprises: cooling means to cool a local region of the patient's skin to selectively interfere with lipid-rich cells in this region, while simultaneously maintaining the patient's skin at a temperature at which the lipid-rich cells are not interfered with, in which the cooling means adapt to the cooling of the lipid-rich cells at a temperature of between -10ºC and approximately 25ºC, a temperature control unit to control the temperature of the cooling means and temperature measurement means that are adapted to measure the patient's skin temperature and/or the temperature in the patient's skin and/or the temperature of the surface of the patient's skin; characterised in that the control unit is also adapted to control the cooling means temperature so that the patients' skin temperature and/or the temperature in the patient's skin and or the temperature of the of the surface of the patient's skin does not fall below a predetermined minimum temperature in function of the patient's skin temperature and/or the temperature in the patient's skin and/or the temperature of the surface of the patient's skin.

Spanish Patent No 2359581 (EP1917935) is also known from the same company, divisional of the previous one, "PROCEDURES AND DEVICES FOR THE SELECTIVE BREAKING UP OF FATTY TISSUE BY MEANS OF CONTROLLED COOLING", from 2003, which refers to a cosmetic treatment procedure for treating a selected region of the abdominal and gluteus regions of human patients, but not babies, in order to achieve a cosmetically beneficial reduction of subcutaneous adipose tissue, which comprises: the use of at least one feedback device to monitor temperature and/or crystal formation, configured to provide feedback information on skin temperature or crystal formation; and to cool the patient's skin based on the information received from at least one feedback device in a region in which a reduction of subcutaneous adipose tissue is desired, by cooling said region sufficiently to selectively break up its lipid-rich cells and to simultaneously maintain the patient's skin at a temperature at which cells not rich in lipids and close to the cooling means do not break up and in which the cells not rich in lipids that surround the subcutaneous fatty tissue do not break up, and in which the lipid-rich cells are cooled to a temperature of between approximately -10ºC and approximately 25ºC for a time period of between 10 seconds and 30 minutes.

It is also worthwhile mentioning French Patent No FR 2613611 "THERMOELECTRIC-EFFECT DEVICE AND ITS CONTROL AND REGULATING MEMBERS, FOR TREATING CANCERS AND OTHER TUMOURS, BY THE METHOD OF ITERATIVE CRYOGENIC APPLICATIONS", from 1987, in the name of Mr Frederic BAUMGARTEN, which refers to a cryogenic apparatus for destroying cancer cells and others by using thermo-electricity, for example, the Peltier effect, characterised in that it comprises: first, regulating members, dc power supply: a refrigerated isothermal annexe, temperature control and a cooling control pump; second, measurement members: dc power supply, a voltmeter with adjustable voltage regulator, voltmeter temperature control and temperature control for the cold probe. It also claims the use of said device for eliminating cancer and other cells by means of cold.

The state of the art also comprises the Spanish Patent No 2010938 "THERMOTHERAPY APPARATUS EMPLOYING COLD", from 1989, in the name of Luis CORRAL SÁNCHEZ, which refers to a thermotherapy apparatus employing cold, fitted with a Peltier cell, located between two thermal plates, one cold and the other receiving heat, with the latter cooled by a refrigerant liquid circuit and also including a drive pump and heat exchanger. The assembly formed by the Peltier cell and the thermal plates constitutes a cold-application head that is independent of the apparatus body.

The closest prior art document is US 2010/0280582 and discloses a head for an apparatus for applying cold-based treatments comprising a chassis with an interior cavity, housing two first cooling conductor plates, a suction orifice connected to suction means, a Peltier cell for each conductor plate, two second conductor plates that dissipate heat towards cooling means, a temperature sensor, power supply means and an extractable accessory with fixing means.

Finally, mention should be made of a book entitled "ELECTRO-AESTHETICS, THEORY AND PRACTICE IN THE USE OF ELECTRIC CURRENTS IN AESTHETICS", BY J. L. VILA BUSQUETS et al, the first edition of which is dated February 1986, in which page 182 describes cryotherapy, vibrotherapy and pressotherapy as aesthetic treatments against OBESITY AND/OR CELLULITIS.

### BRIEF DISCLOSURE OF THE INVENTION

The problem of the prior art is that the head configuration does not take advantage of all the possibilities to increase the cooling surface area so that the required temperature is attained in less time.

Heads are known in aesthetics that absorb the skin after the so-called peeling procedures that equally suck the skin. Heads are known in the state of the art, which employ cryotherapy and vacuum-therapy, cold treatment and suction treatment respectively, but the problems in this case are that not all skins are equally elastic and, depending on the climate, the conditions in the actual treatment room, behaviour may be different so that, if these variables are not taken into consideration, reddening and slight bruising may be caused to the patient.

Thus, the inventor has developed a new head in which an accessory is added to a head, with two facing cooling plates that define a cavity between them and which occupies said cavity in function of the patient's skin so that it reduces the effect of the vacuum-therapy and, at the same time, increases the contact surface with the areas transmitting the cold, without producing any current in the patient's skin.

Said accessory is extractable so that it can be changed for cleanliness purposes as well as for configuring the head cavity in function of the patient to be treated.

One objective of this invention is a head for an apparatus to carry out cold-based treatments, of the type that comprises a chassis, containing a cavity, which houses two first cooling conductor plates that face each other, at least one suction orifice connected to suction means, at least one Peltier cell for each first conductor plate arranged in contact with this and with second heat conductor plates that dissipate heat towards cooling means, a temperature sensor that controls the temperature of the at least one Peltier cell and power supply means, further comprising an extractable, cold-conducting accessory, with fixing means, arranged inside the cavity, reducing the space of said cavity, with said accessory secured inside the cavity by the cited fixing means.

Additionally disclosed wherein is a method for applying locally a cool therapy with the use of the head end, that comprises a chassis and cavity of the type comprising: a first phase, in which the head end is put into operation, cooling said head end by means of its at least two first conductor plates located in the cavity, facing each other, linked to at least one Peltier cell, for each first conductor plate, arranged in contact with this and with second conductor plates that dissipate heat towards cooling means, a second phase that is performed at the same time as the first, or before, or after, in which suction is started inside the cavity of the head end by at least one suction orifice, connected to suction means, characterised in that it comprises: a third phase, in which extractable cold-conducting accessory with fixing means is introduced inside the cavity, reducing the space of said cavity, with said accessory fixed inside the cavity by the referred fixing means, a fourth phase, when cavity is moved closer to the patients' skin, said skin is sucked by the suction means, with said skin being blocked by the accessory, to which, at the same time, it transfers cold, and a fifth phase, in which a temperature sensor monitors the temperature of at least one Peltier cell.

### BRIEF DESCRIPTION OF THE DRAWINGS

In order to facilitate the explanation, it is accompanied by four sheets of drawings that represent a practical exemplary embodiment, which is cited as a non-limiting example of the scope of this invention:
- Figure 1 is a perspective view of the object of this invention seen from below.
- Figure 2 is a section through line II-II of Figure 1, but rotated and seen from below,
- Figure 3 is a close-up view of the cooling means and
- Figure 4 is a perspective view of the accessory.

### SPECIFIC EXECUTION OF THE INVENTION

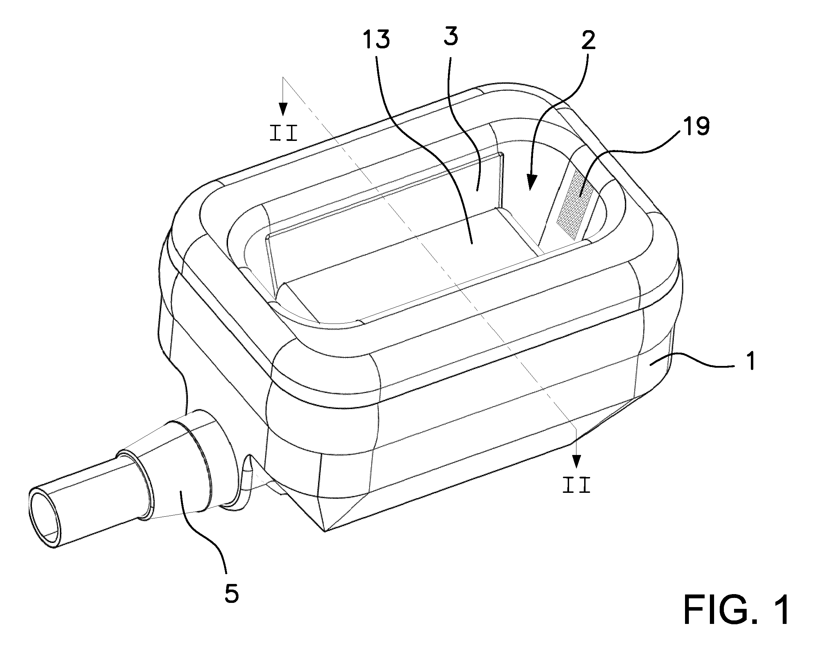

Thus Figure 1 illustrates a chassis 1, a cavity 2, an accessory 13, power supply means 5, a sensor 19, skin-insertion level detector and a first conductor plate 3.

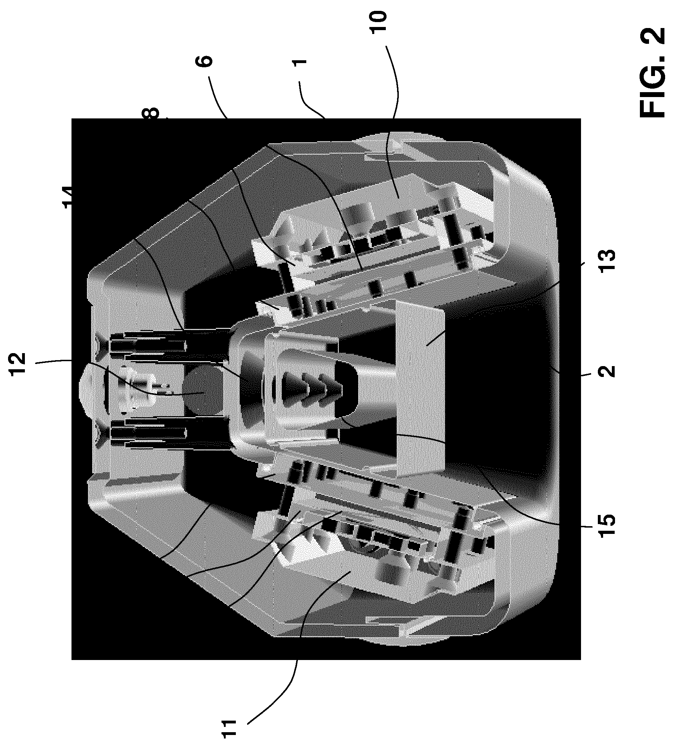

Figure 2 shows chassis 1, cavity 2, accessory 13, its fixing means 14 and its channel 15, first conductor plates 3, 4, Peltier cells 6, 7, second conductor plates 8, 9, cooling means 10, 11 and suction means 12.

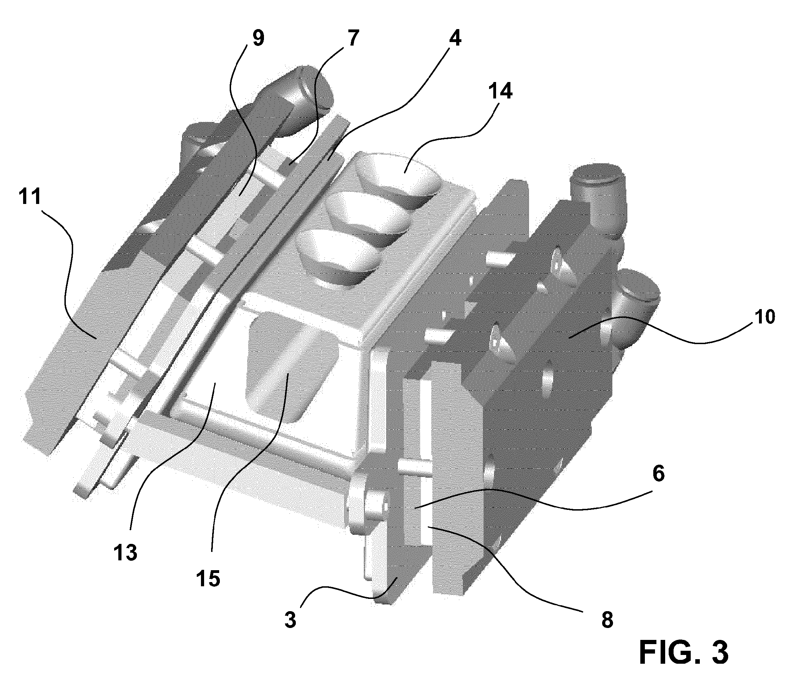

Figure 3 illustrates accessory 13, its fixing means 14 and its channel 15, first conductor plates 3, 4, Peltier cells 6, 7, second conductor plates 8, 9 and cooling means 10, 11.

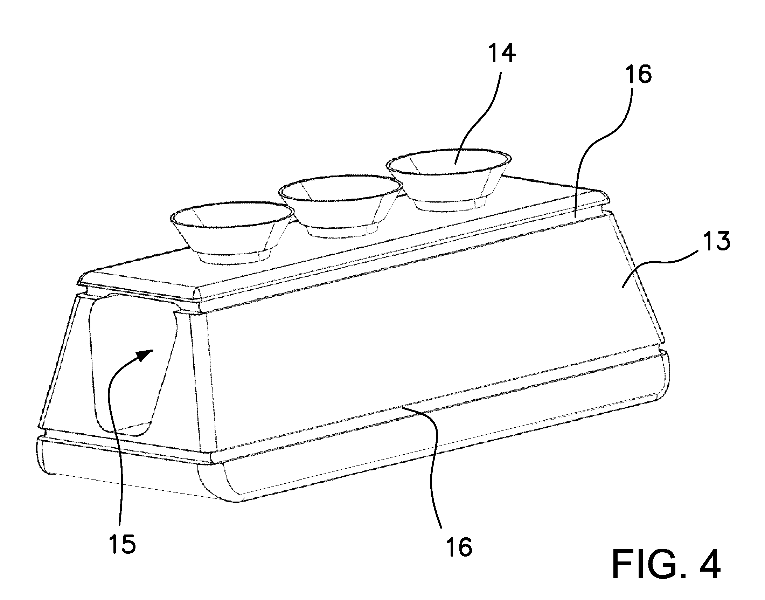

Lastly, Figure 4 shows accessory 13, its fixing means 14, O-rings 16 and its channel 15.

In this way, in a specific embodiment, after preparing the equipment, the operator, depending on the skin type, will position an accessory 13, of one size or another, inside cavity 2, leaving a space between the cavity mouth 2 and accessory 13, which shall be the maximum space that the patient's skin will be sucked inside the head.

When accessory 13 is inserted, it will be secured inside cavity 2 by fixing means 14, for example, suction pads. Accessory 13 shall extractable for cleanliness reasons and so that the correct one may be selected according to the patient's skin.

Then the head is positioned on the part of the patient's body to be treated. Optionally, two or more heads may be employed at the same time, provided the machine allows this.

When the machine is switched on, the operator selects the programme to follow, together with the minutes and temperature.

Power supply means 5, for example, electric, put the head into operation.

Thus, when the current passes through Peltier cells 6, 7, these transform a part into cold that is stored in first conductor plates 3, 4 and the other part in heat which is taken to second conductor plates 8, 9.

Second conductor plates 8, 9 may be in contact with cooling means 10, 11 or, as in this embodiment, form part of cooling means 10, 11. In other words, second conductor plates 8, 9 contain a circuit through which refrigerant liquid flows and limited at the top by cooling means 10, 11, for example, a plastic cover. The refrigerant liquid (not shown) leaves the head by means of pipes and is connected to a drive pump and a heat exchanger, both located outside the head (not shown), which returns the once-again cooled refrigerant liquid to cooling means 10, 11.

On the side of the first conductor plates 3, 4, facing each other, in this embodiment manufactured in a metal conductor, these will be cooled to the required temperature, calculated by a temperature sensor (not shown) located on each first conductor plate 3, 4 and Peltier cells 6, 7 and transmit said cold inside cavity 2.

There may be two Peltier cells 6, 7 on each side, as in Figure 3, or the number may be increased (three or four, etc.) if the head is increased, or reduced if the head is small.

To prevent contact between the patient's skin and the metal of the first conductor plates 3, 4 causing injury to the patient's skin, the said first conductor plates 3, 4 in this embodiment have a ceramic covering.

Moreover, suction means 12 are put into operation and aspirate towards cavity 2. Said suction means comprise an orifice (not shown) that would be arranged at the bottom of cavity 2 and decentred; in this way, the patient's skin does not reach the bottom of cavity 2, if accessory 13 was not present.

Accessory 13 is a conductor of cold, for example, it is manufactured of metal. The cold transmitted by first conductor plates 3, 4 is transmitted towards accessory 13 that is equally cooled.

When the head is positioned on the patient and his skin is sucked inside cavity 2, said skin touches accessory 13, which also includes a ceramic covering, which cools the patient's skin.

Moreover, first conductor plates 3, 4 also cool the patient's skin and so the surface area that cools the patient's skin is greater.

In order to reduce the mass of accessory 13 and increase the cooling speed of accessory 13, together with a reduction in power consumption, accessory 13 is optionally hollow, with channel 15 defined inside it.

In one of the embodiments, cavity 2 has a trapezoid shape and consequently accessory 13 has a trapezoid shape so it can be housed inside, with a larger section at the beginning and becoming progressively narrower as it gains depth.

On the contrary, channel 15 also has a trapezoid shape, but which is inverted in relation to accessory 13. This is to improve thermal inertia. This means that, in the upper part, it is not so necessary for the cold to dissipate because it is not in contact with the patient's skin. On the other hand, this is necessary in the lower part, which means that there is most metal mass in the area closest to the patient's skin.

Accessory 13 may also comprise O-ring 16 outside accessory 13. On the one hand, it aids in fixing accessory 13 inside cavity 2, but, on the other, if the accessory exterior is covered with conductive gel, O-ring 16 forms a barrier so that the gel cannot move towards the patient's skin.

Optionally, the two first conductive cooling plates 3, 4, have a trapezoid shape in order to adapt to the shape of cavity 2 and, in this way, cover the maximum surface area of greatest section at the beginning and smaller as it approaches the bottom of cavity 2.

Moreover, with respect to patient safety, the installation of at least one sensor 19 is contemplated, arranged between the two first conductor plates 3, 4, to detect the level of insertion of the patient's skin inside cavity 2. Thus, if accessory 13 is incorrectly installed, the sensor will provide a warning that too much of the patient's skin is being sucked inside and that the patient is in danger and that the problem can be resolved by changing accessory 13.

Optionally, suction means 12 can produce suction levels of different magnitudes and in a periodic manner via the suction orifice. This is done for two reasons, a first is to massage the skin under suction and the other is to prevent the freezing effect on the skin, where the idea is to move the skin so that it does not freeze.

Thus, head end application method would be as follows.

In a first phase, the head end is switched on, cooling said head end by means of at least two first conductor plates 3, 4 located in the cavity 1, facing each other, just as previously described.

Said first conductor plates 3,4, are linked to at least one Peltier cell 6, 7, for each first conductor plate 3, 4, arranged in contact with this and with second conductor plates 8, 9 that dissipate heat towards cooling means 10,11.

In the second phase that is performed at the same time as the first, or before, or after, suction is started inside the cavity 2 of the head end by at least one suction orifice, connected to suction means 12.

In the third phase, in which extractable cold-conducting accessory 13 with fixing means 14 is introduced inside cavity 2, reducing the space of said cavity 2, with said accessory 13 fixed inside cavity 2 by the referred fixing means 13. In this way, cold from the first conductor plates 3, 4 is transferred to the accessory 13.

In the fourth phase, when cavity 1 is moved closer to the patients' skin, said skin is sucked inside by the suction means 12, with said skin being blocked by the accessory 13, which prevents that moves towards suction means 12 and avoiding causing injury to the skin.

At the same time that the skin is sucked, when the skin touches the accessory 13, the latter cools the skin.

Finally, in the fifth phase, a temperature sensor monitors the temperature of at least one Peltier cell. It is preferred to monitor the Peltier cell temperature, that is not the skin temperature, because this method prevents that a Peltier cell failure can produce an uncontrolled temperature increase that could damage the skin, since such a temperature increase would be immediately detected.

The inventor has observed that, for many skins, the treatment can be carried out below 10ºC. Problems start when certain clinics standardise the processes and this is the reason to decide on a temperature between 10ºC and -5ºC, because most of the skins react within this margin.

Below -5ºC, the inventor has observed that burns may occur, hence the -5ºC is the limit. For example, at -10ºC, pain may occur, together with muscle cramps and even necrosis or fainting.

Treatment is applied during between 35 to 60 minutes, which produces an increase in the blood flow of the area and carries off a large number of frozen cells, for example, fatty cells, from the zone to which the head end is applied.

This invention describes a new head for an apparatus for applying cold-based treatments.

The examples mentioned here do not limit this invention and it could have other applications and/or adaptations, all of which are within the scope of the following claims.

## Claims

1. Head for an apparatus for applying cold-based treatments over patient's skin of the type that comprises chassis (1) with interior cavity (2), which houses the first cooling conductor plates (3, 4), facing each other, at least one suction orifice arranged at the bottom of the cavity and adapted to be connected to suction means (12), at least one Peltier cell (6, 7) for each first conductor plate (3, 4) in contact with this plate and with second conductor plates (8, 9) that dissipate heat towards cooling means (10, 11), a temperature sensor that monitors the temperature of at least one Peltier cell and power supply means (5),
wherein the head further comprises extractable, cold-conducting, skin-contact, accessory (13) with fixing means 14), the accessory being inside the cavity (2) and being placed between the mouth of the cavity (2) and the suction orifice, reducing the interior space of said cavity 2) and leaving a space between the cavity mouth and the accessory (13) wherein said accessory (13) is adapted to be secured inside the cavity (2) by the mentioned fixing means (14) and arranged such that the first conductor plates (3, 4) transmit cold to the accessory (13) that is equally cooled by said transmission of cold.

2. -Head in accordance with claim 1, **characterised in that** accessory (13) is hollow, with channel (15) defined inside it.

3. Head in accordance with claim 2, **characterised in that** cavity (2) has a trapezoid shape and accessory (13) also has a trapezoid shape.

4. Head in accordance with claim 3, **characterised in that** said channel (15) also has a trapezoid shape, but which is inverted in relation to accessory (13).

5. -Head in accordance with one or more of the previous claims, **characterised in that** it comprises at least one O-ring (16) outside accessory (13).

6. Head in accordance with claim 3, **characterised in that** the two first cooling conductor plates (3, 4) have trapezoid shapes.

7. -Head in accordance with claim 1, **characterised in that** it comprises at least one sensor (19), arranged between the two first conductor plates (3, 4), adapted to be a skin-insertion level detector of the patient's skin inside cavity (2).

8. -Head in accordance with one or more of the previous claims, **characterised in that** the two first conductor plates (3, 4), and accessory (13) have ceramic coverings.

9. -Head in accordance with one or more of the previous claims, **characterised in that** the suction orifice is decentred.

10. Head in accordance with claim 9, **characterised in that** suction means (12) is adapted to periodically aspirate different magnitudes through the suction orifice.

## Patentansprüche

1. Kopf für ein Gerät zum Anwenden kältebasierter Behandlungen über der Haut eines Patienten, derart, dass es ein Gehäuse (1) mit innerem Hohlraum (2) umfasst, der die ersten kühlenden Leiterplatten (3, 4) aufnimmt, die sich gegenüber sind, mindestens eine Saugöffnung angeordnet am Boden des Hohlraum und angepasst, mit dem Saugmittel (12) verbunden zu werden, mindestens eine Peltier-Zelle (6, 7) für jede erste Leiterplatte (3, 4) in Kontakt mit dieser Platte und mit zweiten Leiterplatten (8, 9), die Wärme zu Kühlmitteln (10, 11) ableitet, einen Temperaturfühler, der die Temperatur der mindestens einen Peltier-Zelle überwacht, und Stromversorgungsmittel (5), wobei der Kopf ferner ein abnehmbares kälteleitendes Hautkontaktzubehör (13) mit Befestigungsmitteln (14) umfasst, wobei das Zubehör im Hohlraum (2) ist und zwischen der Mündung des Hohlraums (2) und der Saugöffnung angeordnet ist, dabei den Innenraum des Hohlraums (2) verringert und einen Raum zwischen der Mündung der Hohlraums und dem Zubehör (13) belässt, wobei das Zubehör (13) angepasst ist, um im Hohlraum (2) durch die angeführten Befestigungsmittel (14) befestigt zu werden, und so angeordnet ist, dass die ersten Leiterplatten (3, 4) Kälte auf das Zubehör (13), das durch diese Kälteübertragung gleichförmig gekühlt wird.

2. Kopf nach Anspruch 1, **dadurch gekennzeichnet, dass** das Zubehör (13) hohl mit einem darin definierten Kanal (15) ist.

3. Kopf nach Anspruch 2, **dadurch gekennzeichnet, dass** der Hohlraum (2) trapezförmig ist und das Zubehör (13) auch trapezförmig ist.

4. Kopf nach Anspruch 3, **dadurch gekennzeichnet, dass** der Kanal (15) auch trapezförmig ist, was aber in Bezug auf das Zubehör (13) invertiert ist.

5. Kopf nach einem oder mehreren der vorherigen Ansprüche, **dadurch gekennzeichnet, dass** er mindestens einen O-Ring (16) außerhalb des Zubehörs (13) umfasst.

6. Kopf nach Anspruch 3, **dadurch gekennzeichnet, dass** die zwei ersten kühlenden Leiterplatten (3, 4) trapezförmig sind.

7. Kopf nach Anspruch 1, **dadurch gekennzeichnet, dass** er mindestens einen Sensor (19) umfasst, der zwischen den zwei ersten Leiterplatten (3, 4) angeordnet ist, und angepasst ist, ein Graddetektor für Hauteinführung der Haut des Patienten im Hohlraum (2) zu sein.

8. Kopf nach einem oder mehreren der vorherigen Ansprüche, **dadurch gekennzeichnet, dass** die zwei ersten Leiterplatten (3, 4) und das Zubehör (13) keramische Abdeckungen haben.

9. Kopf nach einem oder mehreren der vorherigen Ansprüche, **dadurch gekennzeichnet, dass** die Saugöffnung dezentriert ist.

10. Kopf nach Anspruch 9, **dadurch gekennzeichnet, dass** das Saugmittel (12) angepasst ist, um periodisch verschiedene Größen durch die Saugöffnung anzusaugen.

## Revendications

1. Tête d'un appareil pour appliquer des traitements à base de froid sur la peau d'un patient, du type qui comprend un châssis (1) avec une cavité intérieure (2), où sont logées les premières plaques conductrices de froid (3, 4) l'une en face de l'autre, au moins un trou d'aspiration situé dans la partie inférieure de la cavité et adapté pour son raccordement au moyen d'aspiration (12), au moins un module à effet Peltier (6, 7) pour chaque première plaque conductrice (3, 4) en contact avec cette plaque et avec les deuxièmes plaques conductrices (8, 9) qui envoie la chaleur vers le moyen de refroidissement (10, 11), un capteur de température qui contrôle la température d'au moins un module à effet Peltier et le moyen d'alimentation électrique (5), où la tête comprend, de plus, un accessoire (13) extractible, conducteur du froid, en contact avec la peau, avec un moyen de fixation (14), l'accessoire étant à l'intérieur de la cavité (2), et étant placé entre l'entrée de la cavité (2) et le trou d'aspiration, réduisant l'espace à l'intérieur de ladite cavité (2) et laissant un espace entre l'entrée de la cavité et l'accessoire (13) où ledit accessoire (13) est adapté pour être fixé à l'intérieur de la cavité (2) par ledit moyen de fixation (14) et disposé de telle manière que les premières plaques conductrices (3, 4) transmettent le froid vers l'accessoire (13) qui est également refroidi par ladite transmission de froid.

2. Tête conformément à la revendication 1, **caractérisée en ce que** l'accessoire (13) est creux, un canal (15) y étant défini à l'intérieur.

3. Tête conformément à la revendication 2, **caractérisée en ce que** la cavité (2) a une forme trapézoïdale et que l'accessoire (13) a également une forme trapézoïdale.

4. Tête conformément à la revendication 3, **caractérisée en ce que** ledit canal (15) a également une forme trapézoïdale mais qui est inversé par rapport à l'accessoire (13).

5. Tête conformément à l'une ou à plusieurs des revendications précédentes, **caractérisée en ce qu'**elle comprend au moins un joint d'étanchéité (16) à l'extérieur de l'accessoire (13).

6. Tête conformément à la revendication 3, **caractérisée en ce que** les deux premières plaques conductrices de refroidissement (3,4) ont une forme trapézoïdale.

7. Tête conformément à la revendication 1, **caractérisée en ce qu'**elle comprend au moins un capteur (19) situé entre les deux premières plaques conductrices (3, 4) adapté pour être un capteur de niveau d'insertion dans la peau du patient à l'intérieur de la cavité (2).

8. Tête conformément à l'une ou à plusieurs des revendications précédentes, **caractérisée en ce que** les deux premières plaques conductrices (3, 4) et l'accessoire (13) ont un revêtement céramique.

9. Tête conformément à l'une ou à plusieurs des revendications précédentes, **caractérisée en ce que** le trou d'aspiration est décentré.

10. Tête conformément à la revendication 9, **caractérisée en ce que** le moyen d'aspiration (12) est adapté pour aspirer périodiquement avec différentes magnitudes à travers le trou d'aspiration.
